# EUROPEAN PATENT SPECIFICATION

(11) **EP 2 352 546 B1**
(45) Date of publication and mention of the grant of the patent: **15.01.2014**
(21) Application number: 09814153.4
(22) Date of filing: 17.09.2009
(51) Int. Cl.: A61N 1/18, A61N 1/32, A61N 1/36

(54) **CRANIAL ELECTROSTIMULATION SYSTEM**
SYSTEM ZUR SCHÄDELELEKTROSTIMULATION
SYSTÈME D'ÉLECTROSTIMULATION CRÂNIENNE

(30) Priority: 18.09.2008 GB 0817091
(43) Date of publication of application: 10.08.2011
(73) Proprietor: Brainique AG, 6370 Stans (CH)
(72) Inventor: GREY, Robert, Russell, 8005 Western Cape Province (ZA)
(74) Representative: Lau, Sarah Jane
(86) International application number: PCT/IB2009/006864
(87) International publication number: WO 2010/032112

(56) References cited:
- EP-A2- 0 323 052
- WO-A2-2004/000413
- US-A- 5 458 625
- US-A1- 2007 250 145

## Description

### FIELD OF THE INVENTION

This invention relates to an electrode unit for use in carrying out cranial electrostimulation especially, but not necessarily exclusively, electrostimulation in the form of what is known as cranial electro-biologic stimulation that is typically applied between electrodes clipped to the earlobes of a patient receiving treatment. Such a procedure is often referred to as electrotherapy in many of its applications.

It is to be understood that the term "patient" as used in this specification is intended to be interpreted broadly as being any person being subjected to electrostimulation irrespective of whether the underlying reason for applying such electrostimulation has curative intentions or not. In this regard it is noted that electrostimulation is often used with the intention of simply improving a patient's quality of life such as by improving the patient's mood, emotions, attitude or cognitive capabilities.

### BACKGROUND TO THE INVENTION

Cranial electro-biologic stimulation (herein referred to as CES) applies gentle micro-current pulses to the brain (in the hypothalamic area) using a pair of ear-clip electrodes that attach to the earlobes. It is widely accepted that CES stimulates the brain to manufacture neurotransmitters, like endorphins, which improve moods, emotions and cognitive capabilities. Cranial electrostimulation has also been proposed for treatment following a stroke, brain trauma, certain heart disorders, high blood pressure, jet leg, motion sickness and dementia.

The signals apparently normalise the electrical output of the brain. CES has thus been used/tested to treat insomnia, substance dependence, depression and anxiety. It has been noted in at least some instances that CES has equal or greater efficacy for the treatment of depression when compared to antidepressant medications, with fewer side effects.

The mechanism by which CES produces its effects is not yet fully understood. It is postulated that the stimulation of brain tissue causes increased amounts of neurotransmitters to be released, specifically serotonin, beta endorphin, and noradrenaline. It is believed that these neurotransmitters in turn permit a return to normal biochemical homeostasis of the limbic system of the brain that may have been imbalanced by a stress-related condition.

Treatments that have typically been used range from 10 to 30 minutes in duration although they may extend up to 1 1/2 hours depending on the electrical current configuration. Typically the currents employed would be applied in pulse form with a pulse width in the range of from about 1 to about 500 milliseconds (ms) at a frequency of from about 0.1 Hertz (Hz) to about 500 Hz with the current being typically less than I milliampere (mA) and more typically about 300 to 700 microamperes (µA), although currents of up to about 2.5 milliampere (mA) have also been used.

The beneficial effects of music on the human body have long been appreciated and employed in the treatment, in particular, of psychological disturbances.

Document EP-A-0323052 discloses the most relevant prior art.

### SUMMARY OF THE INVENTION

The invention is defined in claim 1.

Further features of the invention provide for the electrical contact is a contact surface layer exposed for direct contact with the skin of a patient, in use, or alternatively, for the electrical contact to assume the form of an electrically conductive cover for the electrode itself; for the electrode to be carried on an electrically insulating surround to the audio earphone; for the electrode to be in the form of a ring surrounding the electrically insulating surround; and for the electrode unit to be arranged in pairs thereof in which instance the audio earphones may be a pair of stereo earphones of substantially conventional type.

It will be appreciated that the electrode units can be used in carrying out any form of electrostimulation although it is generally envisaged that they will be most suitable for application in carrying out CES. Still more particularly, and whilst not limiting the invention in any way, it is envisaged that the electrode units will, *inter alia,* be particularly useful in the implementation of our co-pending patent application filed contemporaneously herewith under the title "CRANIAL ELECTROSTIMULATION METHOD AND EQUIPMENT" and claiming priority in terms if the International Convention of Paris from British Patent Application No 0817089.6. In that instance the electrostimulation pulses are generated at a frequency that is varied according to the rhythm, beat or tempo of music that is audible to the patient by way of the earphones.

In order that the above and other features of the invention may be more fully understood, different embodiments thereof will be described below with reference to the accompanying drawings.

### BRIEF DESCRIPTION OF THE DRAWINGS

In the drawings:-
- Figure 1: is a block diagram of one method and equipment for applying CES pulses by way of electrode units according to the disclosure;
- Figure 2: is a perspective illustration of a portable version of equipment operating on the basis of the block diagram of Figure 1;
- Figure 3: is a perspective illustration of one embodiment of earphone according to the disclosure and that is suitable for use with the equipment illustrated in Figure 2;
- Figure 4: is an exploded illustration thereof;
- Figure 5: illustrates in Figure 5a an earphone about to be installed in an ear; in Figure 5b the earphone installed; and in Figure 5c the area of contact of the electrode with the ear; and,
- Figure 6: illustrates an alternative form of electrode unit.

### DETAILED DESCRIPTION WITH REFERENCE TO THE DRAWINGS

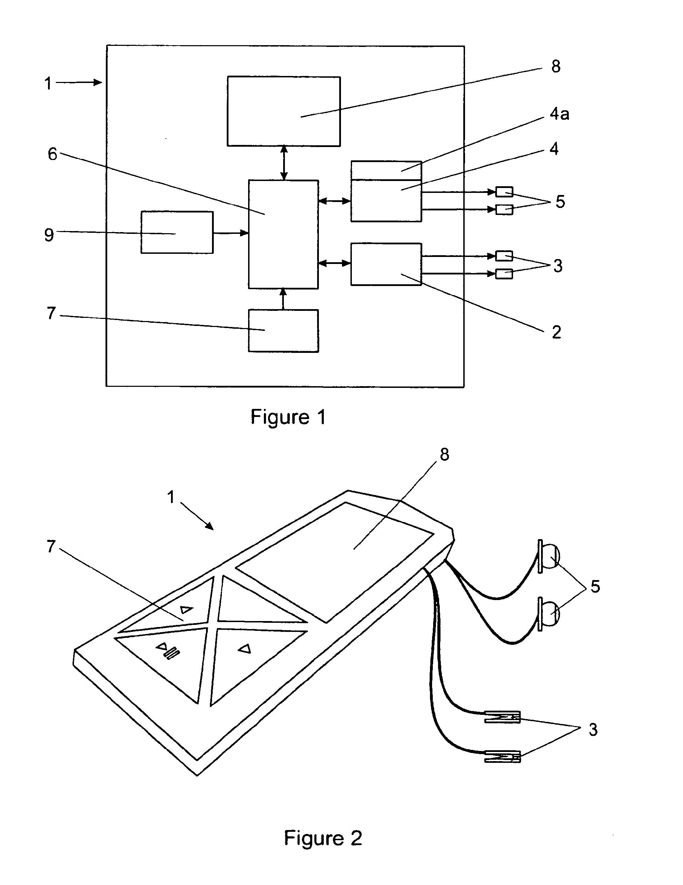

In the equipment illustrated in Figures 1 and 2, a portable battery powered unit (1) is employed to generate CES pulses to carry out CES therapy in a manner as set out in our co-pending patent application indicated above. The equipment may take the shape and configuration of a pocket sized therapy unit as illustrated in Figure 2 or it may be a multi component arrangement such as for use in a treatment centre or studio.

The therapy unit is configured to carry out CES on a patient by the application of appropriate electrical pulses to the ears simultaneously with the playing of music that is audible to the patient. The equipment thus comprises a cranial electrostimulation pulse generator (2) and associated electrodes (3) for applying pulses generated by the pulse generator to the ears of a patient, and a sound signal generator (4) and a pair of earphones (5) for converting output from the sound signal generator into audible sound. The sound signal generator may have associated with it a data base containing a selection of different musical numbers that can be selected by a user or by selecting a predetermined treatment programme or session. The data base is indicated by numeral (4a) in Figure 1.

The outputs from the electrostimulation pulse generator and sound signal generator are controlled by a controller in the form of a microprocessor (6) that may be user operated by a user button interface (7). The unit has a display screen (8) for assisting in programme selection in very much a conventional manner. Obviously the display screen could be replaced by any other suitable indicator means such as an array of light emitting diodes or the like. A battery charger circuit (9) is also provided.

The above arrangement is such that the electrostimulation pulse generator produces electrical pulses having a frequency and pulse width selected to provide a required microcurrent across the head, in use, such microcurrent typically being of the order of 500 to 700 µA and, in any event, less than 1 mA. The frequency could range from 0.5 Hz to about 3 Hz and the pulse width could range from about 125 ms at a frequency of 0.5 Hz to about 75 ms at a frequency of 3 Hz. The microcurrent across the head is, in any event, generally maintained at a substantially constant average current

The pulses (or possibly sequences of pulses in the event that predetermined sequences of optionally different pulses are employed) are, in this instance, generated at a frequency that is dependant on the tempo of music that is being played at any one time and that is audible to the patient by way of the earphones. Of course, in order to achieve a substantially constant average current, the pulse width of the pulses will also vary with variation in frequency. The microprocessor monitors the tempo of the music and controls the generation of the electrical pulses on the basis thereof.

Whilst not in any way being limiting to the scope of the invention it is proposed that the following could operate effectively:-
At an audio tempo of 30 beats per minute a frequency of 0.5 Hz could be employed with a pulse width of 125 ms;
at an audio tempo of 60 beats per minute a frequency of 1.0 Hz could be employed with a pulse width of 115 ms;
at an audio tempo of 90 beats per minute a frequency of 1.5 Hz could be employed with a pulse width of 105 ms;
at an audio tempo of 120 beats per minute a frequency of 2.0 Hz could be employed with a pulse width of 95 ms;
at an audio tempo of 150 beats per minute a frequency of 2.5 Hz could be employed with a pulse width of 85 ms; and,
at an audio tempo of 180 beats per minute a frequency of 3.0 Hz could be employed with a pulse width of 75 ms.

The microprocessor is also programmed to provide treatment programmes or sessions of a predetermined duration, as is well known in the art, and the duration would generally be somewhere between 10 minutes and 30 minutes but may extend up to an hour or even an hour and a half. Information as to any particular treatment session to be chosen, musical numbers and the like can be displayed on the screen (8) provided on the therapy unit.

Reverting now to the nature of the earphones that are provided by this disclosure the electrodes and earphones are combined into combination electrode units that fit into the entrance to the auditory canal of the ear.

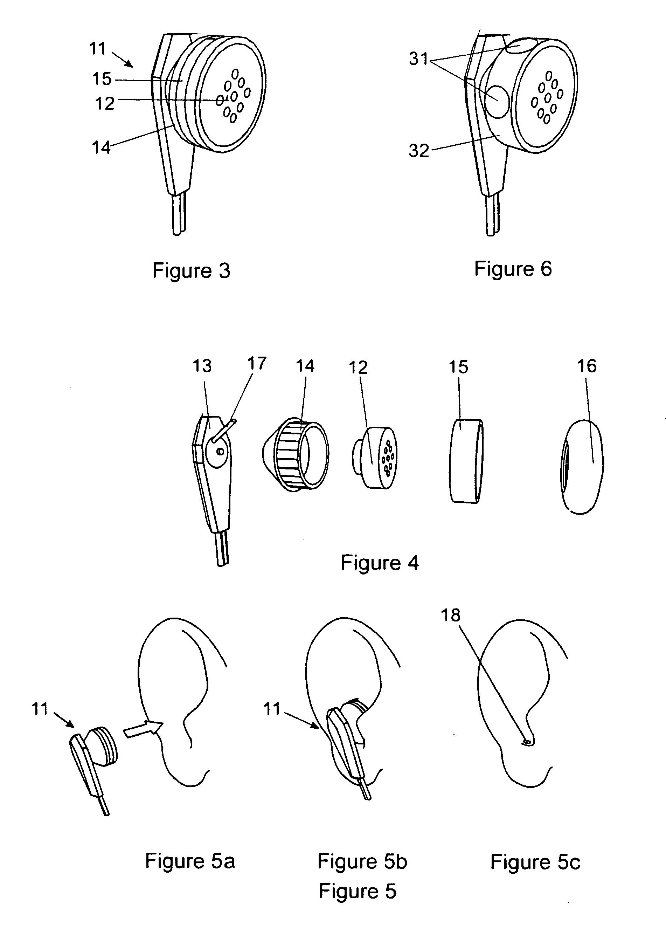

As illustrated in Figures 3 to 5 of the accompanying drawings, each earphone unit (11) may comprise a generally standard audio earphone (12) mounted on a base (13) and surrounded by an insulating ring (14). A ring-shaped electrode (15) surrounds the insulating ring and is covered by an electrically conductive cover in the form of an electrically conductive rubber sheath (16). The electrode is, of course, electrically connected to the electrostimulation pulse generator (2) by way of a suitable conductor (17) so that a current can be generated between the electrodes on two earphones contacting the two ears of a patient, in use.

As shown in Figure 5, the earphone is adapted to be received in the entrance to the auditory canal so that the ring makes connection with the skin by way of the conductive rubber sheath, for example in the region indicated by numeral (18) in Figure 5c. Of course, the earphone could be configured to be received within the auditory canal of the ear in the manner of what are often referred to canal phones or earbuds, as will be quite apparent to those skilled in the art.

Figure 6 illustrates an alternative electrode unit in which the electrode (31) itself is in the form of a single "spot" electrode on a cylindrical insulating surface (32) of the type of earphone adapted to be received in the entrance to the auditory canal.

Of course the electrode unit can be used in association with any other type of CES treatment equipment and is in no way limited to use in association with that described above.

The use of a single electrode unit for both the application of electrical pulses to the ears of a patient and the simultaneous provision of music for a patient, whether or not the music is related in any way to the pulses, is extremely convenient and cost-effective. Such an electrode unit very much simplifies the conduct of electrostimulation accompanied by music for the patient to enjoy whilst undergoing the therapy and also simplifies setting up the equipment on a patient.

It will be understood that numerous variations may be made to the embodiments of the invention described above without departing from the scope hereof.

## Claims

1. A system comprising a pair of stereo audio earphones, each of the earphones comprising an electrode unit (11) for use in cranial electrostimulation, each of the electrode units comprising an electric current transferring electrode (15, 31) for operatively transferring electrical energy to a region of an ear of a patient, the electrode of each electrode unit being carried on the outer periphery of the respective audio earphone (12), each of the audio earphones being of the type suitable for engagement in the entrance to, or within, the auditory canal of an ear of said patient, wherein the electrode of each electrode unit has an electrical contact area for operatively transferring electrical energy between the electrode and the skin of a patient fitted with the electrode unit, wherein said electrical contact area is in the entrance to, or within, the auditory canal of the respective ear of said patient.

2. A system as claimed in claim 1 in which the electrical contact is a contact surface layer exposed for direct contact with the skin of a patient, in use.

3. A system as claimed in claim 1 in which the electrical contact is an electrically conductive cover (16) for the electrode itself.

4. A system as claimed in any one of the preceding claims in which the electrode is carried on an electrically insulating surround (14) to the audio earphone.

5. A system as claimed in claim 4 in which the electrode is in the form of a ring (15) surrounding the electrically insulating surround.

6. A system as claimed in any one of the preceding claims, further comprising an electrostimulation pulse generator, wherein each of the electrodes is connected to the generator by means of a conductor, so that a current is generated between the electrodes on the two earphones contacting the two ears of a patient, in use.

## Patentansprüche

1. System mit einem Paar von Stereo-Ohrhörern, wobei jeder der Ohrhörer eine Elektrodeneinheit (11) zur Verwendung bei elektrischer Schädelstimulation aufweist, wobei jede der Elektrodeneinheiten eine Stromübertragungselektrode (15, 31) zum wirksamen Übertragen von elektrischer Energie an einen Bereich eines Ohrs eines Patienten aufweist, wobei die Elektrode jeder Elektrodeneinheit am Außenumfang des jeweiligen Ohrhörers (12) angebracht ist, wobei jeder der Ohrhörer von der Art ist, die zum Einsetzen im Eingang überhalb des Gehörgangs, eines Ohrs des Patienten geeignet ist, wobei die Elektrode jeder Elektrodeneinheit einen elektrischen Kontaktbereich zum wirksamen Übertragen von elektrischer Energie zwischen der Elektrode und der Haut eines Patienten, der mit der Elektrodeneinheit ausgestattet ist, hat, wobei sich der elektrische Kontaktbereich im Eingang oder innerhalb des Gehörgangs des jeweiligen Ohrs des Patienten befindet.

2. System nach Anspruch 1, wobei der elektrische Kontakt eine Kontaktoberflächenschicht ist, die zum direkten Kontakt mit der Haut eines Patienten im Gebrauch freigelegt ist.

3. System nach Anspruch 1, wobei der elektrische Kontakt eine elektrisch leitende Beschichtung (16) für die Elektrode selbst ist.

4. System nach einem der vorhergehenden Ansprüche, wobei die Elektrode an einer elektrisch isolierenden Umfassung (14) des Ohrhörers angebracht ist.

5. System nach Anspruch 4, wobei die Elektrode in Form eines Rings (15) vorliegt, der die elektrisch isolierende Umfassung umgibt.

6. System nach einem der vorhergehenden Ansprüche, das des Weiteren einen Elektrostimulationsimpulsgenerator aufweist, wobei jede der Elektroden mittels eines Leiters mit dem Generator verbunden ist, so dass zwischen den Elektroden an den beiden Ohrhörern, die mit den beiden Ohren eines Patienten in Kontakt stehen, im Gebrauch Strom erzeugt wird.

## Revendications

1. Système comprenant une paire d'écouteurs audio stéréo, chacun des écouteurs comprenant une unité d'électrode (11) pour leur utilisation dans une électrostimulation crânienne, chacune des unités d'électrode comprenant une électrode (15, 31) de transfert de courant électrique pour transférer fonctionnellement une énergie électrique à une région d'une oreille d'un patient, l'électrode de chacune des unités d'électrode étant portée sur le pourtour extérieur de l'écouteur audio respectif (12), chacun des écouteurs audio étant du type approprié pour être engagé dans l'entrée du conduit auditif ou dans le conduit auditif d'une oreille dudit patient, dans lequel l'électrode de chaque unité d'électrode a une zone de contact électrique pour le transfert fonctionnel d'une énergie électrique entre l'électrode et la peau d'un patient doté de l'unité d'électrode, dans lequel ladite zone de contact électrique est dans l'entrée du conduit auditif ou dans le conduit auditif de l'oreille respective dudit patient.

2. Système selon la revendication 1, dans lequel le contact électrique est une couche de surface de contact exposée pour un contact direct avec la peau d'un patient lors de l'utilisation.

3. Système selon la revendication 1, dans lequel le contact électrique est une couverture électriquement conductrice (16) pour l'électrode elle-même.

4. Système selon l'une quelconque des revendications précédentes, dans lequel l'électrode est portée sur un encadrement électriquement isolant (14) de l'écouteur audio.

5. Système selon la revendication 4, dans lequel l'électrode a la forme d'un anneau (15) entourant l'encadrement électriquement isolant.

6. Système selon l'une quelconque des revendications précédentes, comprenant en outre un générateur d'impulsion d'électrostimulation, dans lequel chacune des électrodes est raccordée au générateur au moyen d'un conducteur, de sorte qu'un courant soit généré entre les électrodes sur les deux écouteurs en contact avec les deux oreilles d'un patient, lors de l'utilisation.
